# EUROPEAN PATENT APPLICATION

(11) **EP 4 083 193 A1**
(43) Date of publication of application: **02.11.2022**
(21) Application number: 20907774.2
(22) Date of filing: 21.12.2020
(51) Int. Cl.: C12N 5/076, A01K 67/02, A01N 1/00, A01K 67/027

(54) **RAT SPERM FREEZING METHOD AND IN VITRO FERTILIZATION METHOD**

(30) Priority: 23.12.2019 JP 2019231190
(71) Applicant: National University Corporation Kumamoto University, Kumamoto-shi, Kumamoto 860-8555 (JP)
(72) Inventor: NAKAGATA, Naomi, Kumamoto-shi, Kumamoto 860-8555 (JP); TAKEO, Toru, Kumamoto-shi, Kumamoto 860-8555 (JP); MIKODA, Nobuyuki, Tosu-shi, Saga 841-0075 (JP)
(74) Representative: Parchmann, Stefanie
(86) International application number: PCT/JP2020/047732
(87) International publication number: WO 2021/132176

(57) **Abstract**

An object of the present invention is to provide a method for cryopreserving rat sperms and an in vitro fertilization method using cryopreserved rat sperms. The present invention provides a method for preparing cryopreserved rat sperms, characterized by comprising the following steps: step a: a preparation step of collecting rat sperms from the rat cauda epididymis to prepare a sperm suspension, step b: a cooling step of cooling the sperm suspension to about 1°C or lower, and step c: a freezing step of freezing the rat sperm suspension cooled to about 1°C or lower.

## Description

### [Technical Field]

The present invention relates to a method for cryopreserving rat sperms. The present invention also relates to an in vitro fertilization method using rat sperms cryopreserved using the method.

### [Background Art]

Over the last 10 years, gene-deficient or introduced transgenic rats and base-replaced point-mutated rats have been produced in large numbers throughout the world. In recent years, genome editing technology has come to be generally used in the research field of rats as well. Therefore, it is predicted that more genetically modified rats will be produced by genome editing technology in the near future. However, since rats are about 10 times larger than mice, there is a limit to the number of rats that can be bred in experimental animal facilities. As a result, many genetically modified rat strains have been conserved in rat banks (Rat Resource Center) as gametes or cryopreserved embryos. Typical rat banks include the National BioResource Project-Rat at Kyoto University in Japan, the Rat Resource & Research Center at University of Missouri in the United States, and the Gene Editing Rat Resource Center at Medical College of Wisconsin in the United States.

As one of the methods for preserving rat strains, there is a method for cryopreserving rat embryos. In the method of cryopreserving rat embryos, since 400 to 500 embryos are preserved per strain, it is necessary to collect embryos from the oviducts of 30 to 50 mating females. The number of females that can be mated at one time is limited, and males must be mated with females many times, so if males with some genetic modifications are used for mating, it takes 2 to 4 months to obtain the quantity of embryos sufficient for cryopreservation. On the other hand, in the method of cryopreserving rat sperms, it is possible to cryopreserve a huge number of sperms immediately after being collected from the epididymis of a male. Therefore, for the preservation of genetically modified rat strains, cryopreservation of sperms is much easier, more efficient and economically superior than cryopreservation of embryos.

In vitro fertilization using fresh rat sperms was demonstrated by Toyoda et al. in 1974, but for a considerable period of time thereafter, no successful in vitro fertilization using frozen rat sperms has been reported. The first report on cryopreservation of rat sperms was published in 2001 by Nakatsukasa et al. (Non-Patent Document 1: Nakatsukasa et al., Reproduction 122, 463-467 (2001)). Although many reports have been published since then, only a report by Seita et al. has reported that pups were obtained from embryos obtained by in vitro fertilization using frozen rat sperms (Non-Patent Document 2: Seita et al., Biol. Reprod. 80, 503-510 (2009)). They reported that when frozen/thawed sperms treated with IBMX were used for in vitro fertilization, the proportions of pronucleus formation and blastocyst formation significantly increased (pronucleus formation rate: 50%; blastocyst formation rate: 20%; birth rate: 49%) as compared with the case of use of frozen/thawed sperms not treated with IBMX, however, it is not a practical technique because the fertilization rate is low. Since this study, there have been no reports showing frozen sperms that retain sufficient motility to produce pups by in vitro fertilization using frozen sperms and embryo transfer of fertilized oocytes. Therefore, a satisfactory protocol for freezing rat sperms and a satisfactory in vitro fertilization protocol using frozen rat sperm by said protocol have not been established so far.

### [Citation List]

Non-Patent Document 1: Nakatsukasa et al., Reproduction 122, 463-467 (2001))
Non-Patent Document 2: Seita et al., Biol. Reprod. 80, 503-510 (2009)
Non-Patent Document 3: Nakatsukasa et al., Comp. Med. 53, 639-641 (2003)

### [Summary of the Invention]

### [Technical Problem]

An object of the present invention is to provide a method for cryopreserving rat sperms and a method for in vitro fertilization using cryopreserved rat sperms.

### [Solution to Problem]

Rat sperms are known to be very sensitive to environmental changes such as the viscosity of the solution in which the rat sperms are placed and osmotic stress. The commonly used rat sperm cryopreservation solution (CPA) has higher viscosity and osmotic pressure (370 to 380 mOsm) than the viscosity and osmotic pressure (300 to 310 mOsm) of the diluted solution mTHF. Therefore, the present inventors considered that if a cryopreservation solution (CPA) containing sperms is diluted with a diluent immediately after thawing, the cryopreserved sperms may be possibly damaged. Therefore, in order to solve the above-mentioned problem, the present inventors have diligently studied a method for reducing the environmental changes in the situation where the rat sperms are placed, and resultantly found that a higher fertilization rate and pup developing ability can be achieved in in vitro fertilization as compared with the conventional case by freezing rat sperms by a specific step and/or thawing frozen rat sperms by a specific step, leading to completion of the present invention.

The present invention includes the followings.

### (Cryopreservation method)

[1] A method for preparing cryopreserved rat sperms, characterized by comprising the following steps:
   Step a: A preparation step of collecting rat sperms from the rat cauda epididymis to prepare a sperm suspension,
   Step b: A cooling step of cooling the sperm suspension to about 1°C or lower (preferably about 0.5°C or lower, more preferably about 0°C), and
   Step c: A freezing step of freezing the rat sperm suspension cooled to about 1°C or lower.
[2] The method according to [1] above, wherein the cooling step of said step b is performed in order to substantially reduce the movement of rat sperms.
[3] The method according to [1] or [2] above, wherein said step b comprises a two-stage process of (b-1) a step of cooling the rat sperm suspension to about 4°C to about 6°C (preferably about 4°C to about 5°C) (hereinafter, referred to as primary cooling of the step b in some cases), and (b-2) a step of further cooling the primarily cooled sperm suspension to about 1°C or lower (preferably about 0.5°C or lower, more preferably about 0°C) (hereinafter, referred to as secondary cooling of the step b in some cases).
[4] The method according to [3] above, wherein at least said step (b-2) is performed in a cryopreservation container for cryopreserving sperms (preferably in a straw-shaped cryopreservation container).
[5] The method according to [3] or [4] above, wherein said step (b-2) is performed by placing the cryopreservation container containing the sperm suspension on ice for about 15 minutes to about 45 minutes (preferably about 25 minutes to about 35 minutes, more preferably about 30 minutes).
[6] The method according to any one of [1] to [5] above, wherein in said step b, the motion capability of the sperms after cooling is 20% or less (preferably 15% or less, more preferably 10% or less, further preferably 5% or less, still further preferably 2% or less) as compared with the motion capability of the sperms before cooling.
[7] The method according to any one of [1] to [5] above, wherein in said step b, the motion rate of the sperms after cooling is 50% or less (preferably 40% or less, more preferably 30% or less, further preferably 20% or less) as compared with the motion rate of the sperms before cooling.
[8] The method according to any one of [1] to [7] above, wherein said rat cauda epididymis is rat cauda epididymis stored under refrigeration.
[9] The method according to any one of [1] to [8] above, wherein said rat sperm is a sperm derived from a gene-modified rat.
[10] Use of the cryopreserved rat sperms prepared by the method as described in any one of [1] to [9] above, for in vitro fertilization.

### (Freezing and thawing method)

[11] A method for preparing rat sperms for use in in vitro fertilization, characterized by comprising the following steps:
   Step A: A thawing step of heating a cryopreservation solution containing frozen rat sperms to a temperature of about 35°C to about 37.5°C (preferably about 36°C to about 37.5°C, more preferably about 37°C) to prepare a thawed rat sperm suspension,
   Step B: A swim-up step of placing said thawed rat sperm suspension at a lower portion of the medium contained in a container and allowing it to stand to swim up the sperms,
   Step C: A first recovery step of recovering sperms, and
   Step D: A second recovery step of transferring the recovered sperms to a sperm culture solution and recovering sperms with high motility.
[12] The method according to [11] above, wherein the lower portion of the container in said step B is a tube having a tapered or conical tip.
[13] The method according to [11] or [12] above, wherein the medium in said step B has a volume of about 5 to about 20 times that of the sperm suspension.
[14] The method according to any one of [11] to [13] above, wherein the standing in said step B is conducted for about 20 to about 40 minutes.
[15] The method according to any one of [11] to [14] above, wherein the step C is a step of recovering sperms by mixing the thawed rat sperm suspension and the medium in a container and then centrifuging at a low speed to recover the precipitate.
[16] Use of the rat sperms prepared by the method as described in any one of [11] to [15] above, for in vitro fertilization.

### (In vitro fertilization method)

An in vitro fertilization method, comprising the following steps:
   (i) a step of thawing the cryopreserved rat sperms prepared by the method as described in any one of [1] to [9] above, using the method as described in any one of [11] to [15] above, and preparing them for in vitro fertilization, and then pre-culturing the prepared rat sperms in a sperm preculture medium (here, the sperm preculture medium is also a medium used for in vitro fertilization in the step (iii)),
   (ii) a step of administering horse chorionic gonadotropin (eCG) to female rats and then human chorionic gonadotropin (hCG), and then collecting unfertilized oocytes to prepare unfertilized oocytes, and
   (iii) a step of mixing the sperms pre-cultured in the step (i) with the unfertilized oocytes prepared in the step (ii) to perform insemination.
The in vitro fertilization method according to [17] above, wherein said step (ii) further includes a cumulus removal step of removing the cumulus oophorus of the prepared unfertilized oocyte.
The in vitro fertilization method according to [17] or [18] above, wherein the sperm preculture medium in said step (i) is an mHTF medium containing about 1 to about 80 mg/mL (preferably about 4 to about 80 mg/mL, more preferably about 10 to about 60 mg/mL, further preferably about 20 to about 60 mg/mL, still further preferably about 30 to about 50 mg/mL) of bovine serum albumin.

### (Sperm preculture medium)

A rat sperm preculture medium, which is an mHTF medium or TYH medium containing about 20 mg/mL to about 80 mg/mL (preferably about 20 to about 60 mg/mL, more preferably about 30 to about 50 mg/mL) of bovine serum albumin, used for pre-culturing rat sperms, in in vitro fertilization using cryopreserved rat sperms.

### (Fertilization medium)

A rat in vitro fertilization medium for use in in vitro fertilization using cryopreserved rat sperms, which is an mHTF medium or TYH medium containing about 20 to about 80 mg/mL (preferably about 20 to about 60 mg/mL, more preferably about 30 to about 50 mg/mL) of bovine serum albumin.

One embodiment of the present invention relates to rat sperm cryopreservation, which provides a rat sperm freezing method that can achieve better fertilization rate and/or pup developing ability than the prior art, when conducting in vitro fertilization using rat sperms after freezing-thawing.

Another embodiment of the present invention relates to thawing of frozen rat sperms, which provides a frozen rat sperm thawing method that can achieve better fertilization rate and/or pup developing ability than the prior art, when conducting in vitro fertilization using rat sperms after thawing.

Another embodiment of the present invention relates to in vitro fertilization using frozen rat sperms, which combines the method of freezing rat sperms and the method of thawing frozen rat sperms of the present invention to provide a rat sperm in vitro fertilization method that can achieve better fertilization rate and/or pup developing ability than the prior art.

### [Advantageous Effect of the Invention]

The method for cryopreserving rat sperms and/or the method for thawing frozen rat sperms of the present invention provides in vitro fertilization using frozen rat sperms capable of achieving better fertilization rate and/or pup developing ability than the prior art.

### [Brief Description of Drawings]

FIG. 1 is a schematic view of a method for cryopreserving rat sperms, which is one embodiment of the present invention.
FIG. 2 is a schematic view of a method for thawing frozen rat sperms, which is one embodiment of the present invention.
FIG. 3 is a view showing an embodiment of an instrument that can be used in the method for freezing rat sperms of the present invention. The figure shows the instrument viewed from diagonally above.
FIG. 4 is a view of the cryopreservation container placement rack as viewed from diagonally above used for the freezing aid instrument shown in FIG. 3.
FIG. 5 is a view of the cryopreservation container placement rack as viewed from above used in the freezing aid instrument shown in FIG. 3.
FIG. 6 is a view of the cryopreservation container placement rack as viewed from side used for the freezing aid instrument of FIG. 3.
FIG. 7 is a view showing another embodiment of the instrument that can be used in the method for freezing rat sperms of the present invention.
FIG. 8 is a view of the cryopreservation container placement rack as viewed from diagonally above used for the freezing aid instrument shown in FIG. 7.
FIG. 9 is a view of the cryopreservation container placement rack as viewed from above used in the freezing aid instrument shown in FIG. 7.
FIG. 10 is a view of the cryopreservation container placement rack as viewed from side used in the freezing aid instrument shown in FIG. 7.

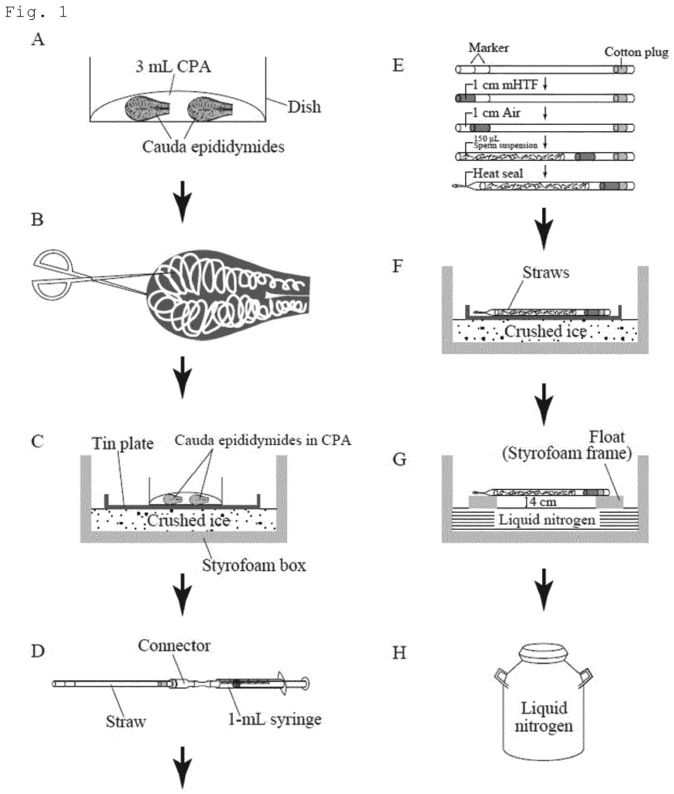

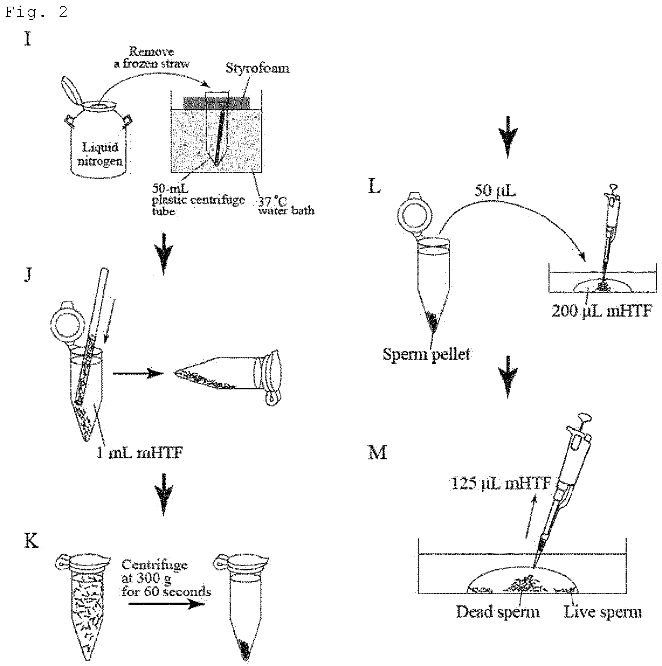

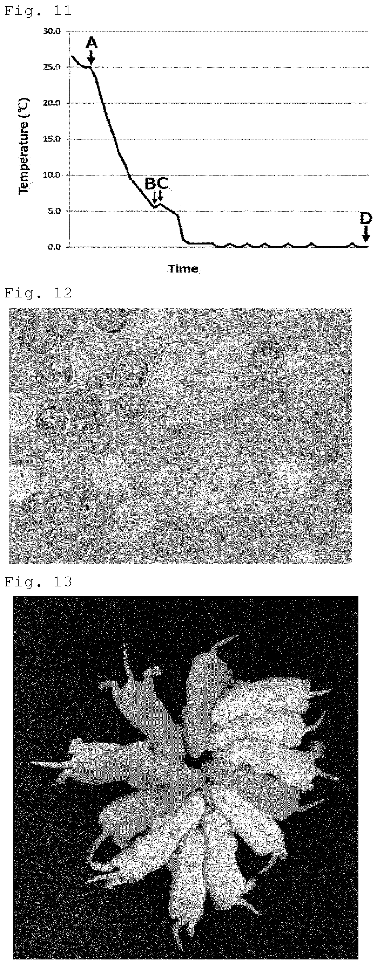

FIG. 11 is a view showing a temperature change of a sperm suspension in a step of freezing rat sperms by the rat sperm freezing method of the present invention. In the figure, each operation process is shown together with the drawing number of each process shown in FIGS. 1 and 2. A indicates the time when the sperm suspension is started to cool on ice (C in FIG. 1), B indicates the time when the sperm suspension is filled in the straw (D in FIG. 1), C indicates the time when the straw is started to cool on ice (F in FIG. 1), and D indicates the time when the straw is started to freeze in liquid nitrogen (G in FIG. 1).
FIG. 12 is a microscope-observed photograph of a blastocyst stage embryo obtained by in vitro fertilization using rat sperms cryopreserved and thawed using the method of the present invention. Embryos that are green (look faint in black and white image) are embryos that emit a GFP signal.
FIG. 13 is a photograph of pups born by embryo transfer to rat of a fertilized oocyte obtained by in vitro fertilization using rat sperms cryopreserved and thawed using the method of the present invention. Green (looks white in black and white image) pups is pups that emits a GFP signal.
FIG. 14 shows the results of observing sperm motility after preserving the rat cauda epididymis with a Lifor^{®} refrigeration preservation solution supplemented with various concentrations of dimethyl sulfoxide (DMSO) and quercetin (Q). The figure on the left shows the proportion of sperms in movement 2 hours after the preparation of the sperm suspension, and the figure on the right shows the proportion of sperms in movement 6 hours after the preparation.
FIG. 15 shows a photograph of an oocyte prepared by a conventional method (untreated oocyte: left figure) and a photograph of an oocyte from which cumulus oophorus has been further removed (cumulus oophorus-removed oocyte: an oocyte from which surrounding cumulus cells have been removed: right figure).

### [Description of Embodiments]

Hereinafter, the present invention will be illustrated with reference to the exemplary embodiments and is not intended to be limiting to the following embodiments.

Unless otherwise specified in the sentences, any technical terms and scientific terms used in the present specification have the same meaning as those generally understood by those of ordinary skill in the art to which the present invention belongs. Any materials and methods equivalent or similar to those described in the present specification can be used for practicing the present invention.

All publications and patents cited herein in connection with the present invention described herein are incorporated by reference, for example, as indicating methodology, materials, etc. that can be used in the present invention.

In the present specification, when the expression "X to Y" is used, it means that X is included as a lower limit and Y as an upper limit, or that X is included as an upper limit and Y as a lower limit. In the present specification, "about" is used to mean that ±10% is allowed.

The present inventors were able to cryopreserve transgenic rat sperms by using the method of the present invention described below, and succeeded in in vitro fertilization with an oocyte using the cryopreserved sperms. In addition, normal pups were developed from oocytes fertilized in vitro.

### [Rat sperm cryopreservation method]

One embodiment of the present invention is a method for cryopreserving rat sperms, which comprises the following steps.
Step a: A preparation step of collecting rat sperms from the rat cauda epididymis to prepare a sperm suspension.
Step b: A cooling step of cooling the sperm suspension to about 1°C or lower, and
Step c: A freezing step of freezing the rat sperm suspension cooled to about 1°C or lower.

The outline of the cryopreservation method of rat sperms is shown in FIGS. 1A to 1H.

### (1) Step of preparing sperm suspension

In the method for cryopreserving rat sperms of the present invention, a rat sperm suspension is first prepared. Rat sperms are collected from the rat cauda epididymis and suspended in a cryopreservation solution according to a conventional method to prepare a sperm suspension. As the cryopreservation solution (CPA), a cryopreservation solution that can be used for preserving sperms of animals, preferably small animals, can be used with appropriate modifications as necessary. Alternatively, a commercially available cryopreservation solution may be used.

The method for preparing a rat sperm suspension is not limited to this, but for example, it can be preferably carried out as follows. The cauda epididymis is collected from euthanized mature male rats. The collected cauda epididymis is placed in a CPA drop (1 cauda epididymis/1.5 mL) prepared in the center of a petri dish at room temperature (20 to 25°C), and scissors or a scalpel make multiple cuts, preferably about 10 times, on the cauda epididymis, to prepare a sperm suspension. Then, the petri dish is placed on a metal plate having excellent thermal conductivity (for example, a tin plate) placed on crushed ice, and preferably left for about 10 minutes or longer to cool the sperm suspension. This cools the sperm suspension to about 4°C to about 6°C.

The cauda epididymis containing sperms stored under refrigeration after being collected from a rat may also be used. For example, cauda epididymis can be collected from euthanized mature male rats, placed in a refrigeration preservation solution, and preserved under refrigeration (e.g., at about 4°C) until sperm suspension preparation. Examples of the preservation solution include, but are not limited to, Lifor^{®} (Lifeblood Medical Inc.) preservation liquid, which is a refrigeration preservation solution for human organs. The Lifor^{®} preservation liquid is an artificial preservation solution containing nutrients, growth factors, and carriers for non-proteinaceous enzymes and nutrients. Preferable is a preservation solution in which dimethyl sulfoxide (DMSO) and/or quercetin is further added to Lifor^{®}. The concentration of dimethyl sulfoxide added to the preservation solution is usually 1 to 30 %, preferably 5 to 25 %, and more preferably 5 to 20%. The concentration of quercetin added to the preservation solution is usually 10 to 1000 µg/mL, preferably 50 to 250 µg/mL, and more preferably 50 to 200 µg/mL. The refrigerated preservation period is usually 7 days or less, preferably 5 days or less, and more preferably 3 days or less. In addition, since it can be preserved under refrigeration, it can be transported in a refrigerated state.

### (2) Cooling step to cool rat sperm suspension to about 1°C or lower

The method for cryopreserving rat sperms of the present invention is characterized by including a cooling step of cooling the rat sperm suspension to about 1°C or lower (however, the rat sperm does not freeze) before freezing. By this cooling step, the rat sperm suspension is cooled to about 1°C or lower, preferably about 0.5°C or lower, more preferably about 0°C, to a temperature at which the rat sperm does not freeze. This cooling step can substantially reduce the movement of rat sperms in the suspension. By substantially reducing the movement of rat sperms, damage to rat sperms can be reduced in cryopreservation including subsequent freezing steps, and a good cryopreserved rat sperm suspension can be prepared. If the rat frozen sperms thus prepared are then thawed and used for in vitro fertilization, the rat frozen sperms can attain improved fertilization rate, as compared with sperms treated by a conventional method, that is, sperms obtained by cooling rat sperms in a cryopreservation solution on a petri dish on ice, then, transferring into a cryopreservation container, and immersing into liquid nitrogen to be cryopreserved.

The step of cooling a rat sperm suspension to about 1°C or lower preferably includes, after cooling the sperm suspension to about 4°C to about 6°C (preferably about 4°C to about 5°C) (hereinafter, referred to as "primary cooling" in some cases), further cooling to about 1°C or lower (hereinafter, referred to as "secondary cooling" in some cases). However, it may be continuously cooled to about 1°C or lower without being divided into two stages, which is also included in the method of the present invention. The method for primarily cooling the sperm suspension is not limited, but, for example, a method of placing a petri dish containing a sperm suspension on ice, preferably, a method of laying a metal plate having good thermal conductivity, for example, a tin plate, on ice and placing a petri dish on it so that the bottom of the petri dish is uniformly cooled can be used. Alternatively, a method of immersing a container (for example, a tube) containing a sperm suspension into a solution cooled to about 4°C to cool the suspension, or a method of placing a tube or petri dish containing a sperm suspension into a constant temperature cooling tank or a cooling box set at about 4°C to cool the suspension can be used.

As the method of cooling the primarily cooled sperm suspension to about 1°C or lower, for example, a method of transferring a primarily cooled sperm suspension to a cryopreservation container, and then cooling the sperm suspension in the cryopreservation container to about 1°C or lower can be mentioned. The cryopreservation container is not particularly limited as long as it is a container that can be used for cryopreservation of rat sperms, and examples thereof include a straw-shaped container and a tube-type container.

When the cauda epididymis preserved under refrigeration is used, a sperm suspension may be prepared, cooled, and then subjected to the above steps, alternatively a series of operations for sperm suspension preparation can be performed at a low temperature (for example, about 4°C). For example, the sperm suspension can be prepared by placing a petri dish on a metal plate with excellent thermal conductivity (e.g., a tin plate) placed on crushed ice, placing the cauda epididymis in a cooled (e.g., cooled to about 4°C) CPA drop made at the center of the petri dish (1 cauda epididymis/1.5 mL), and making multiple cuts, preferably about 10 times, using scissors or a scalpel in the cauda epididymis. When a series of sperm suspension preparation operations are performed at a low temperature using the cauda epididymis preserved under refrigeration, the above-mentioned primary cooling step may be omitted.

The secondary cooling is not limited, but preferably, a method of transferring the primarily cooled sperm suspension into a straw-shaped cryopreservation container and placing it again on ice, preferably on a metal plate having good thermal conductivity (for example, a tin plate) placed on ice and cooling it to about 1°C or lower can be mentioned. The time of placing on ice or on a metal plate is not particularly limited as long as the sperm suspension drops to about 1°C or lower, but for example, when a straw-shaped container is used, it is about 15 minutes to about 45 minutes, preferably about 20 minutes to about 40 minutes, more preferably about 25 minutes to about 35 minutes, further preferably about 30 minutes. Here, "on ice or on a metal plate" means to place a cryopreservation container (for example, a straw-shaped container) directly on ice or a metal plate. This causes the sperm suspension to cool more uniformly.

Further, for the secondary cooling of a sperm suspension, a container containing a sperm suspension may be placed in ice. Alternatively, the secondary cooling of a sperm suspension can be performed by using a method of placing a cryopreservation container containing a sperm suspension in a constant temperature cooling box set at about 0°C and cooling the sperm suspension. When using a cooling box with a program, a cryopreservation container containing a sperm suspension can be placed in the cooling box, and the primary cooling and the secondary cooling can be continuously performed.

For the step b, any of the above-mentioned methods can be used, but preferably, it is a method in which a petri dish containing a sperm suspension is placed on a metal plate laid on ice and cooled to about 4°C to about 6°C, and then the sperm suspension is transferred to a straw-shaped cryopreservation container, it is placed again on a metal plate on ice and cooled to about 1°C or lower.

The sperm suspension can be inserted into the straw-shaped cryopreservation container by using a known method reported. For example, a procedure can be mentioned in which a straw-shaped container with a major axis outer diameter of 1.9 mm (inner diameter 1.6 mm, length 133 mm) is used, 30 µL of an mHTF solution is aspirated carefully into the straw with 10 mm of air, then 150 µL of a sperm suspension is aspirated, and further, the plunger of the syringe is pulled up until 30 µL of the mHTF solution reaches the cotton plug of the straw, and finally the tip of the straw is sealed with an impulse sealer.

In the step b, rat sperms are cooled to about 1°C or lower to substantially reduce sperm movement. In this step, "substantially reduce sperm movement" means that the motion capability of the sperms after cooling is about 20% or less, preferably about 15% or less, more preferably about 10% or less, further preferably about 5% or less, still further preferably about 2% or less, and most preferably about 1% or less, as compared with the motion capability before cooling the sperms as an index. Here, the sperm motion capability means the ability of the sperms to move, and the sperms having the motion capability is, for example, not limited to, (1) a sperm that goes straight at a high speed, (2) a sperm that goes straight but at a low speed, (3) a sperm that moves fast but does not go straight, (4) a sperm that rotates on the spot, and (5) a sperm that has head or tail movement but do not move whole body. Therefore, to compare the motion capability, for example, the number of sperms (1) to (5) above is counted using the sperm suspension before the cooling step, the number of sperms (1) to (5) above is counted using the sperm suspension after cooling to about 1°C or lower, and these are compared.

The above requirements (1) to (5) indicating the state of sperms having motion capability can be arbitrarily selected and combined. For example, since most of the cooled rat sperms are mainly classified into (4) or (5), the ratio of the number of sperms of (1) to (3) (number of sperms of [(1) to (3)]/number of sperms of [(1) to (5)] × 100%) is obtained using the sperm suspension before the cooling step and the sperm suspension after cooling to about 1°C or lower, and these are compared. Therefore, "substantially reducing sperm movement" means that the ratio of the number of sperms of (1) to (3) above after cooling is about 20% or less, preferably about 15% or less, more preferably about 10% or less, further preferably about 5% or less, still further preferably about 2% or less, and most preferably about 1% or less as compared with that before cooling the sperms. When the rat sperms are cooled by using the conventional method, that is, when the sperm suspension contained in the tube is cooled on ice, the motion capability of the rat sperms is only lowered to about 20% to about 30% compared to before cooling if the ratio of the number of sperms of (1) to (3) above (number of sperms of [(1) to (3)]/number of sperms of [(1) to (5)] × 100%) is adopted as an index, however, in the method of the present invention, the rat sperms are cooled to about 1°C or lower, so that the movement of the rat sperms is further suppressed to about 20% or less as compared with that before cooling.

In the present invention, another index of "substantially reduce sperm movement" is that the motion rate of sperms after cooling is about 50% or less, preferably 40% or less, more preferably about 30% or less, further preferably about 20% or less, and most preferably about 10% or less, as compared with the motion rate before cooling the sperms. The motion rate of sperms is the ratio of the number of sperms in movement to the total number of sperms, and is, not limited to, for example, the ratio of sperms corresponding to (1) to (5) above with respect to the whole sperm. Therefore, the sperms moving after cooling is about 50% or less, preferably about 40% or less, more preferably about 30% or less, further preferably about 20% or less, and most preferably 10% or less as compared with that before cooling. When rat sperms are cooled using conventional methods, that is, when the sperm suspension contained in a tube is cooled on ice, the motion rate of rat sperms is only lowered to about 50% to about 60% as compared with that before cooling if the ratio of the number of sperms in movement relative to the total sperm number is adopted as an index, however, in the method of the present invention, the rat sperms are cooled to about 1°C or lower, so that the movement of the rat sperms is further suppressed and it is about 50% or less as compared with that before cooling. The motion rate of rat sperms before cooling is usually about 60% to about 70%. Therefore, "substantially reduce sperm movement" means, in other words, that the motion rate of sperms after cooling (the ratio of the number of sperms in movement to the total number of sperms) is about 35% or less, preferably about 30% or less, more preferably about 20% or less, further preferably about 15% or less, and most preferably about 10% or less with respect to the whole sperm.

### (3) Freezing step of freezing rat sperm suspension

As the freezing step, a method of immersing a cryopreservation container containing a sperm suspension cooled to about 1°C or lower in liquid nitrogen and freezing it can be mentioned. The freezing method can be carried out by using a known method, but more preferably, in the method of the present invention, a freezing container containing a sperm suspension, preferably a straw-shaped freezing container, is placed on a styrene foam frame, immediately floated on liquid nitrogen in the styrene foam box and left for any time, for example, about 10 minutes or longer, to freeze the sperms. After that, the freezing container is transferred to a dedicated cassette, immersed in liquid nitrogen in the tank, and preserved until use. Rat sperms cryopreserved in this way are thawed at the time of use and used for in vitro fertilization.

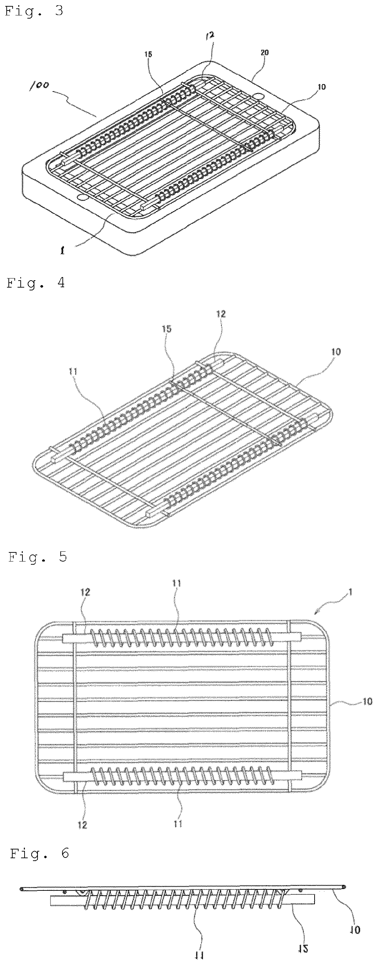

Hereinafter, the instrument that can be preferably used in the freezing step of the method of the present invention will be described. FIG. 3 shows one embodiment of a freezing aid instrument that can be used in the method for freezing rat sperms of the present invention.

Hereinafter, the freezing aid instrument will be described with reference to FIGS. 3 to 7. The instrument shown in the figure is a freezing aid instrument 100 for freezing a rat sperm suspension placed in a straw-shaped cryopreservation container 15. The freezing aid instrument comprises a cryopreservation container placement rack 1 and a float portion 20 on which the cryopreservation container placement rack can be fixed. In the cryopreservation container arrangement table 1, cryopreservation container holding portions 11 for arranging a straw-shaped cryopreservation container (hereinafter, simply referred to as "preservation container") 15 are arranged on the left and right on the main body pedestal portion 10. The main body pedestal portion 10 is provided with a space portion so that cold air from liquid nitrogen, which is a cooling source arranged under the main body pedestal portion, can efficiently pass through. The material of the main body pedestal portion is not particularly limited, but is preferably composed of a member having good thermal conductivity, for example, a metal. In the figure, the main body pedestal portion is composed of a plurality of metal rods, but the number is not limited as long as the preservation container holding portion can be arranged, and may be composed of, for example, only the circumferential portion. The preservation container holding portion 11 is not particularly limited as long as it can hold the preservation container and can form an appropriate distance between the liquid nitrogen as a cooling source and the preservation container. In FIG. 3, a coil-shaped member in which the rod 12 is arranged is shown, but the present invention is not limited to this. The bar may be of any shape, but is preferably a round bar or a square bar. The material of the coiled member and the rod is not particularly limited. When the preservation container is placed on it, the preservation container is held by a coil and a rod placed therein, and an appropriate distance can be formed between the cooling source and the preservation container. Freezing can be performed more slowly than when the preservation container is transferred to a dedicated cassette after cooling and immersed in liquid nitrogen in the tank. This can reduce the damage to sperms.

As shown in FIG. 3, the cryopreservation container placement rack, on which the preservation container holding portions for holding the straw-shaped cryopreservation containers are arranged on the left and right, is fixed on a float portion for floating on liquid nitrogen. The straw-shaped cryopreservation container containing the sperm suspension is placed on the preservation container holding portion of the cryopreservation container placement rack fixed on the float portion, so that it does not move freely even in the state in which the freezing aid instrument is floated on liquid nitrogen. The cryopreservation aid instrument is floated on liquid nitrogen and the sperm suspension is frozen. The frozen sperm suspension is then transferred to a tank of liquid nitrogen and preserved.

FIG. 4 is a view of the cryopreservation container palacement rack used for the freezing aid instrument shown in FIG. 3 as viewed from diagonally above. FIG. 5 is a view of the cryopreservation container palacement rack used for the freezing aid instrument shown in FIG. 3 as viewed from above. FIG. 6 is a view of the cryopreservation container palacement rack used for the freezing aid instrument of FIG. 3 as viewed from side.

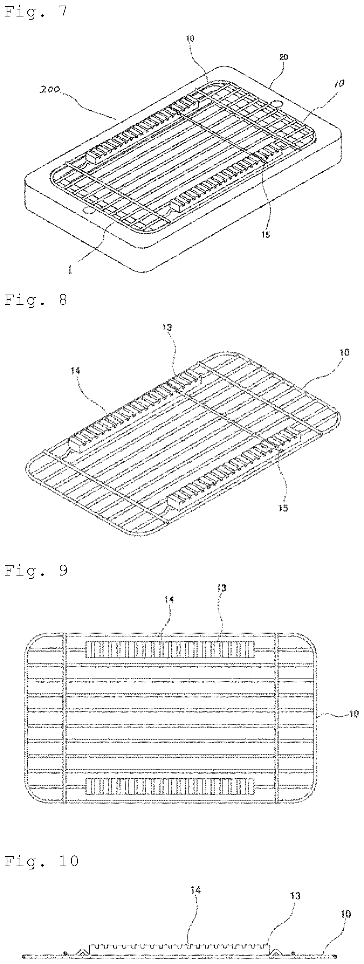

FIGS. 7 to 10 are views showing another embodiment of the instrument that can be used in the method for freezing rat sperms of the present invention. In FIG. 7, the preservation container holding portion 13 is composed of an elongated pedestal-shaped member, and the pedestal is provided with a small notch 14 so that the preservation container does not roll. FIG. 8 is a view of the cryopreservation container palacement rack used for the freezing aid instrument shown in FIG. 7 as viewed from diagonally above. FIG. 9 is a view of the cryopreservation container palacement rack used for the freezing aid instrument shown in FIG. 7 as viewed from above. FIG. 10 is a view of the cryopreservation container palacement rack used for the freezing aid instrument of FIG. 7 as viewed from side.

Therefore, the instruments that can be used in the freezing step of the cryopreservation method of the present invention are as follows.
(1) An instrument used for freezing sperms (sperm freezing aid instrument), which consists of a float portion 20 for floating on liquid nitrogen and a cryopreservation container palacement rack 1 arranged or fixed on the float portion, in which the cryopreservation container palacement rack has a main body pedestal portion 10 which is substantially square or substantially rectangular (wherein, the main body pedestal portion has at least partially penetrating space portion), and at least two preservation container holding portions 11 placed on the main body pedestal portion (wherein, the preservation container holding portion has a mechanism for holding a straw-shaped preservation container and limiting free movement thereof).
(2) The sperm freezing aid instrument according to (1) above, wherein the main body pedestal portion is composed of a plurality of metal rods.
(3) The sperm freezing aid instrument according to (1) or (2) above, wherein the space portion of the pedestal portion constitutes half or more, preferably 70% or more, more preferably 80% or more, further preferably 90% or more of the pedestal portion.
(4) The sperm freezing aid instrument according to any one of (1) to (3) above, wherein the two preservation container holding portions are arranged facing each other near the outer periphery of the main body pedestal portion.
(5) The sperm freezing aid instrument according to any one of (1) to (4) above, wherein the preservation container holding portion has a structure composed of a coil-shaped member and a square bar or a round bar arranged therein.
(6) The sperm freezing aid instrument according to any one of (1) to (4) above, wherein the preservation container holding portion is composed of an elongated pedestal-shaped member having a notch in a part of the surface.

### [frozen rat sperm thawing method]

Another embodiment of the present invention is a method for thawing frozen rat sperms, which comprises the following steps. Thawed rat sperms can then be used for in vitro fertilization. The outline of the method for thawing frozen rat sperms is shown in FIGS. 21 to M.
Step A: A thawing step of preparing a thawed rat sperm suspension by heating a cryopreservation solution containing rat sperms to a temperature of about 35°C to about 37.5°C and thawing it.
Step B: A swim-up step of placing the thawed rat sperm suspension at the lower portion of the medium contained in a container and allowing it to stand for sperm swim-up.
Step C: A first recovery step of recovering sperms, and
Step D: A second recovery step of transferring the recovered sperms to a culture solution and recovering sperms having high motility.

### <1> Step of preparing thawed rat sperm suspension

In the frozen rat sperm thawing method of the present invention, first, frozen rat sperms are thawed to prepare a thawed rat sperm suspension. The frozen preservation solution containing rat sperms is thawed according to a conventional method to prepare a rat sperm suspension. Frozen rat sperms are thawed by heating the frozen sperm suspension to a temperature of about 35°C to about 37.5°C, preferably about 36°C to about 37.5°C, more preferably about 37°C. A container containing a frozen sperm suspension, preferably a straw-shaped container, but not limited to, is placed in a water bath at 37°C and pre-incubated to thaw the sperm suspension. The incubation time in a water bath is not particularly limited as long as it is thawed, but is, for example, about 5 minutes to about 30 minutes, preferably about 10 minutes to about 20 minutes, and more preferably about 15 minutes.

### <2> Sperm swim-up step

The frozen rat sperm thawing method of the present invention is characterized by comprising a swim-up step of placing the thawed rat sperm suspension at the lower portion of the medium and allowing the sperms to swim up . Here, placing at the lower portion of the medium means placing the sperm suspension in a place located at the lower part of or below the medium. The method of placing the sperm suspension under the medium is not particularly restricted, but is, for example, not limited to, a method in which a suitable amount of the medium is applied in a tube, preferably a tube having a tapered or conical tip (for example, an Eppendorf tube or a culture tube), then the thawed sperm suspension can be placed at the bottom of the tube using a narrow tube or tip. Alternatively, the sperm suspension is placed at the bottom of the tube and the medium is slowly layered thereon, thus, the sperm suspension can be placed at the lower portion of the medium. A preferred method is to place the sperm suspension at the bottom of the tube containing the medium. Since the density of the sperm suspension is higher than the density of the medium, the sperm suspension stays at the bottom of the tube. As a result, an environment is formed in which the sperm suspension and the medium are in contact with each other with a boundary surface, and the healthy sperms swim up from the suspension to the medium. The ratio of the amount of the thawed sperm suspension to the amount of the medium is not particularly limited as long as an environment in which the sperms can swim up can be formed, but the amount of the medium is preferably about 5 to 20 times, more preferably about 5 times to about 15 times, further preferably about 5 times to about 10 times the amount of the sperm suspension. By taking such a swim-up step, sperms can be gradually transferred from the environment of the cryopreservation solution to the environment of the culture medium for in vitro fertilization. As a result, the degree of change in the environment is extremely small as compared with the conventional method of adding the thawed sperm suspension to the culture medium and mixing. The standing time for sperms swimm-up is not particularly limited, but is, for example, about 10 minutes to about 60 minutes, preferably about 20 to about 40 minutes, and more preferably about 25 minutes to about 35 minutes.

The medium for sperms to swim up out of the sperm suspension is not particularly limited as long as it is a medium that can be used for sperm culture (sperm culture medium), and examples thereof include an mHTF medium and a TYH medium, and commercially available medium can be used without limitation. Preferably, bovine serum albumin (BSA) is added to the medium, in an amount of preferably about 1 to about 80 mg/mL, more preferably about 4 to about 80 mg/mL. The fertilization rate can be increased by adding BSA.

### <3> Sperm recovery step (first recovery step)

After acclimatizing the sperms to the environment of the culture medium by the sperm swim-up step, the sperms are recovered. Preferably, the sperm suspension and the culture medium are mixed prior to recovering the sperms, preferably mixing is performed slowly to homogenize the medium. For example, this can be done by slowly flipping the tube several times or by slowly inverting the tube to mix. The method for recovering sperms from the culture medium is not particularly limited, and sperms can be recovered, for example, by centrifuging at a low speed (for example, about 200 to about 500 g, preferably about 200 to about 400 g), for about 30 seconds to about several minutes, preferably about 60 seconds. After centrifugation, the suspension containing sperm pellets gathered at the bottom of the tube can be recovered, for example, using a pipette equipped with a pipette tip having wide tip diameter (Large Orifice Tip).

### <4> Sperm recovery step (second recovery step)

The sperms recovered in the first recovery step are transferred to the sperm culture medium (sperm preculture medium) again, and the motile sperms are recovered according to a conventional method. For example, the suspension containing the sperm pellets recovered in the first recovery step is transferred to a drop of a sperm preculture medium covered with a paraffin solution placed in a petri dish, and sperms are cultured. Highly motile sperms swim the periphery of the drop, while less motile sperms gather in the center of the drop. The volume of the drop of the culture medium is not particularly limited as long as the sperms can be cultured and the sperms having high motility and the sperms having low motility can be separated, but is, for example, about 100 µL to about 300 µL, preferably about 150 µL to about 250 µL, more preferably about 200 µL. The medium to be used is not particularly limited as long as it is a medium that can be used for culturing sperms (sperm culture medium), and examples thereof include an mHTF medium and a TYH medium, and commercially available medium can be used without limitation. Preferably, bovine serum albumin (BSA) is added to the medium, in an amount of about 1 to about 80 mg/mL, preferably about 4 to about 80 mg/mL, more preferably about 10 to about 60 mg/mL, further preferably about 20 to about 60 mg/mL, still further preferably about 30 to about 50 mg/mL. The fertilization rate can be increased by adding BSA. After culturing (swimming) the sperms in the drop for about 20 to about 40 minutes, preferably about 25 to about 35 minutes, more preferably about 30 minutes, the highly motile sperms are recovered. Recovery of highly motile sperms may be performed by recovering sperms having high motility swimming the periphery of the drop with a pipette or the like, or by removing sperms with low motility with a pipette or the like. Then, the sperms recovered in the second recovery step is pre-incubated in a medium and then inseminates an oocyte.

### [In vitro fertilization method]

Another embodiment of the present invention is a method of performing in vitro fertilization using sperms cryopreserved by the method of cryopreserving sperms of the present invention. Another embodiment of the present invention is a method of performing in vitro fertilization using sperms thawed using the method of thawing frozen sperms of the present invention. Another embodiment of the present invention is a method of performing in vitro fertilization using sperms cryopreserved by the method of cryopreserving sperms of the present invention and further using sperms thawed using the method of thawing frozen sperms of the present invention.

The in vitro fertilization method can be carried out referring to known reports, for example, the report by Nakagata et al. (Jikken Dobutsu. 41, 443-447 (1992)) and the report by Anzai et al. (Jikken Dobutsu. 43, 445-448 (1994).). For example, it can be performed by the following steps.
(i) A step of pre-culturing rat sperms in a sperm preculture medium,
(ii) A step of administering horse chorionic gonadotropin (eCG) to female rats and then human chorionic gonadotropin (hCG), and then collecting unfertilized oocytes to prepare unfertilized oocytes, and
(iii) A step of mixing the sperms precultured in the step (i) with the unfertilized oocytes prepared in the step (ii) in a medium to perform insemination.

As the in vitro fertilization medium (sperm preculture medium) used in the step (iii), for example, an mHTF medium can be mentioned, and known or commercially available fertilization medium can be used without limitation. Preferably, bovine serum albumin (BSA) is added to the medium, in an amount of about 1 to about 80 mg/mL, preferably about 4 to about 80 mg/mL, more preferably about 10 to about 60 mg/mL, further preferably about 20 to about 60 mg/mL, and most preferably about 30 to about 50 mg/mL. The fertilization rate can be increased by adding BSA.

As the unfertilized oocyte prepared in the above step (ii), an oocyte from which the cumulus oophorus has been further removed can also be used. The removal of the cumulus oophorus can be performed by appropriately referring to known methods. For example, it can be removed by enzymatic treatment with 0.05 to 0.5% hyaluronidase. By using the oocytes from which the cumulus oophorus has been removed, an improvement in fertilization rate can be achieved in in vitro fertilization of rats of a low fertilization rate, for example, rats of Long-Evans, BN or F344 strain.

### EXAMPLES

Hereinafter, the present invention will be specifically described with reference to examples, but the present invention is not limited to the following examples.

### 1.1. Material and method

### (1) Animal

As sperm donors, 11 to 12 week old male transgenic rats (SD-Tg (CAG-EGFP) 40sb) purchased from Slc Japan (Shizuoka, Japan) (http://www.anim.med.kyoto-u.ac.jp/NBR/strains/Strains d.aspx?StrainID=559 & s_geneAffected=GFP & s_References=GFP & s_livingAnimals=1) were used. As oocyte donors, 4 to 5 week old SD female rats purchased from Slc Japan were used. On the other hand, as recipients of fertilized oocytes, 10 to 15 week old Crl: CD (SD) female rats were purchased from Charles River Japan (Kanagawa, Japan). All animals were bred under conditions of a 12-hour light-dark cycle (bright period from 7:00 to 19:00), room temperature of 22 ± 1°C, and constant feeding of feed and water. All animal experiments were conducted with the approval of the Experimental Animal Committee of Kumamoto University School of Medicine.

Wister, Long-Evans, BN, and F344 strain rats were purchased from Charles River Japan (Kanagawa, Japan) and used, in addition to SD strain rats, to examine differences in fertilization rates among various rat strains. In the following examples, SD strain rats were used unless the rat strain was specified.

### (2) Sperm cryopreservation solution (Sperm Cycroprotective Agent: Sperm CPA)

Preparation of the sperm cryopreservation solution (CPA) was basically performed according to the method reported by Nakatsukasa et al. (Non-Patent Document 1: Nakatsukasa et al., Reproduction 122, 463-467 (2001), Non-Patent Document 3: Nakatsukasa et al., Comp. Med. 53, 639-641 (2003)). A solution of 8% (w/v) lactose and 23% (w/v) chicken oocyte yolk to which 1,000 units/mL penicillin G and 1 mg/mL streptomycin sulfate were added was mixed with distilled water. After adjusting the pH of the mixed solution to 7.4 with a 10% trisaminomethane solution, the solution was centrifuged at 1,600 G for 15 minutes and the upper layer was recovered. Next, 0.7% Equex Stm (ES : Nova Chemical Sales, Inc., USA) and 0.1% ATP (Adenosine 5'-triphosphate disodium salt hydrate) were added to the solution and mixed on a stirring plate. Finally, CPA was dispensed in fixed portions and preserved at -20°C until use.

### (3) Sperm cryopreservation

One transgenic rat was used for each experiment for cryopreservation of sperms. The outline of the cryopreservation step is shown in FIGS. 1A to 1H. The cryopreservation step using the fresh cauda epididymis was performed as follows.
1. Male rats were euthanized by cervical dislocation, after which, the two cauda epididymis were aseptically removed and transferred to a 300 µL CPA drop placed in a 35 mm petri dish at room temperature (FIG. 1A).
2. Using scissors under a microscope, 10 to 12 deep cuts were made in the epididymis (FIG. 1B).
3. The petri dish on which the CPA drop was placed was placed on a tin plate placed on crushed ice and allowed to stand for 10 minutes (FIG. 1C).
4. While the sperms were equilibrated in the CPA, the connector at the tip of a 1 mL syringe was attached to the end of the straw, which is a cryopreservation container (FIG. 1D), and placed on the tin plate described above for cooling. Fifteen straws were prepared for two cauda epididymis of one male.
5. At 0°C, 30 µL of mHTF (Kyudo Co., Ltd., Japan) was carefully aspirated into the straw with 10 mm of air. Then, 150 µL of sperm suspension was aspirated and the syringe plunger was pulled up until 30 µL of mHTF reached the cotton plug of the straw. Next, the tip of the straw was sealed using an impulse sealer (FIG. 1E).
6. Fifteen sealed straws were placed on a tin plate placed on crushed ice and allowed to stand for 30 minutes (FIG. 1F).
7. The straw was transferred to a float (styrene foam frame), and then the styrene foam frame holding the straw was immediately floated on liquid nitrogen in the styrene foam box and placed there for 10 minutes (FIG. 1G).
8. After 10 minutes, the straw was immersed in liquid nitrogen. The straws were then transferred to a triangular cassette and preserved in a tank for 4 to 8 weeks (FIG. 1H).

### (4) Sperm cryopreservation using refrigerated cauda epididymis

One transgenic rat was used for each experiment for cryopreservation of sperms. First, male rats were euthanized by cervical dislocation, then, the two cauda epididymis were aseptically removed and transferred into a refrigeration preservation solution contained in a tube (Lifor^{®} refrigeration preservation solution supplemented with dimethyl sulfoxide and quercetin). Then, it was cooled to about 4°C. It was preserved in a cooled state for a certain period (1 to 11 days). The cauda epididymis was then removed from the tube and transferred into a 300 µL CPA drop placed in a 35 mm petri dish. Hereinafter, the above steps 2 to 8 were carried out to prepare frozen sperms.

### (5) Thawing of frozen sperm

Of the 15 cryopreservation straws, 3 to 4 straws were used in the sperm thawing experiment for each experiment. The outline of the thawing step is shown in FIGS. 21 to M. The thawing step was carried out as follows.
1. One milliliter (1 mL) of mHTF was placed in a 1.5 mL volume tube and the medium was equilibrated in an incubator (37°C, 5% CO₂) for 30 minutes prior to use.
2. The cryopreservation straw was removed from the liquid nitrogen tank, placed in a floating container, and then pre-incubated in a water bath at 37°C for 15 minutes to thaw (FIG. 21).
4. The straw was removed from the water bath and the surface of the straw was wiped with a paper towel.
5. The straw was cut in the area between the sperm suspension and the seal and placed in a tube containing the mHTF prepared in 1. Next, after cutting the end of the cotton plug, the straw was inserted into the straw connector and the plunger of the 1 mL syringe was pushed down to transfer only the sperm suspension to the bottom of the tube. Then, the tube was laid on its side and held in a humidifying incubator (37°C, 5% CO₂) for 30 minutes (FIG. 2J) .
6. After 30 minutes, the tube was slowly inverted 2 to 3 times to mix and centrifuged at 300 g for 60 seconds (FIG. 2K). Next, using a 200 µL pipette (catalog number 4290-00S, Funakoshi Co., Ltd., Japan) equipped with a pipette tip having wide tip diameter (Large Orifice Tip), 50 µL of the precipitate containing sperm pellets was aspirated from the bottom of the tube. The precipitate was transferred to an mHTF drop containing 200 µL of BSA (1 to 80 mg/mL) covered with a paraffin solution placed on a petri dish (FIG. 2 L).
7. After 30 minutes, 125 µL of the medium containing dead sperms was removed from the mHTF drop (FIG. 2M) and the sperm suspension was pre-incubated for 2 hours prior to insemination.

### (6) Sperm preculture medium (in vitro fertilization medium)

As the sperm preculture medium (in vitro fertilization medium), modified human tubal fluid (mHTF) containing BSA (4 mg/mL or 40 mg/mL) was used. The prepared medium was sterilized by filtration through a filter (0.22 µm), then, it was preserved at 4°C.

### (7) In vitro fertilization and development of fertilized oocyte

In each experiment, in vitro fertilization was performed using cryopreserved sperms and fresh sperms collected from 4 male rats. For cryopreserved sperms, the sperm suspension having total motility of 30% and progressive motility over 10% as measured by computer-assisted sperm analysis (Integrated Visual Optical System, Hamilton Thorn Inc. USA) prior to insemination was used for in vitro fertilization.

In vitro fertilization was carried out according to the procedure described in the report by Nakagata et al. (Jikken Dobutsu. 41, 443-447 (1992)) and the report by Anzai et al. (Jikken Dobutsu. 43, 445-448 (1994).). Briefly, it is as follows.

Juvenile female rats were induced to ovulate by administering 30 IU of horse chorionic gonadotropin (eCG: Aska Pharmaceutical) and 30 IU of human chorionic gonadotropin (hCG, Aska Pharmaceutical) at intervals of 54 to 56 hours. Fifteen to 16 hours after administration of hCG, the rats were euthanized by cervical dislocation and the oviduct was promptly collected. All intact cumulus oophorus oocyte complexes were separated from the collected oviduct and placed in the pre-incubated sperm suspension (sperm concentration: 500 to 1200 cells/µL) (insemination). As a control, hyperovulatory oocytes obtained using the same method were introduced into a fresh sperm suspension (sperm concentration: 500 cells/µL) incubated for 2 hours. Twenty hours after insemination, the oocytes were observed under an inverted microscope, and the fertility rate was calculated by dividing the number of fertilized oocytes (if two pronuclei are found in the cytoplasm or if the two pronuclei are fused and only the sperm tail can be confirmed, these are judged to be fertilized oocytes) by the total number of oocytes mixed with the sperms and multiplying by 100.

In the test with cumulus-removed oocytes, the intact cumulus oophorus oocyte complexes were isolated from the oviduct and transferred to a medium containing 0.1% hyaluronidase and treated with an enzyme for 1 to 3 minutes, then, washed with the medium. Then, in an in vitro fertilization medium, a pre-incubated sperm suspension (sperm concentration: 500 to 1200 cells/µL) and oocytes were mixed (insemination). Hereinafter, the fertilization rate was calculated in the same manner as described above.

In each experiment, 20 fertilized oocytes were transplanted to the oviduct of a Crl: CD (SD) female rat on the day when the vaginal plug was seen (the first day of pseudopregnancy) by embryo transfer via the wall of the oviduct, at 10 embryos/oviduct. After 22 to 23 days, the number of pups was recorded. The rest of the fertilized oocytes were cultured for an additional 8 hours, and 100 fertilized oocytes developed to the 2-cell stage were cultured in mRlECM until the blastocyst stage. The GFP signal in the developed blastocyst was observed with a fluorescence microscope.

### (8) Introduction of GFP gene

For production of introduced genes, chicken beta-actin promoter, cytomegalovirus enhancer, green fluorescent protein (EGFP) and Rabbit beta globin PolyA were introduced into the pCAGGS vector developed by Niwa et al. (Niwa et al, Gene, 1991, 108, 193-9) based on the pUC13 plasmid, to prepare linear DNA cleaved with BamHI and Sal 1. A solution of the linear DNA fragment was injected into the pronucleus of a fertilized oocyte collected from an SD rat and transplanted into the oviduct of a pseudo-pregnant female rat. By irradiating the pups obtained by the above operation with ultraviolet rays, green fluorescence was detected, and an individual into which the GFP gene was introduced was detected. Heterozygous rats were produced by mating GFP rats and SD rats, and the strain was maintained by mating the heterozygous male rats and SD female rats.

### (9) Statistical analysis

Statistical analysis was performed using Prism version 5.0 (GraphPad). The results are described as mean ± standard deviation SD. Group results were compared using analysis of variance after percentage arcsine transformation. p <0.05 was determined to be statistically significant.

### (10) Evaluation of sperm motion capability

The pre-cultured sperms are diluted with mHTF and the sperms are introduced into a dedicated chamber (Hamilton Thorne, Inc.). After that, these are set in HTM-IVOS (Hamilton Thorne, Inc.) and the sperm motion capability can be analyzed. The ratio (%) of sperms having motion capability (Motile sperm) can be shown, for example, as the ratio of sperms moving 5 µm or more per second to the total number of sperms. The ratio (%) of sperms having progressive motion capability (progressive motile sperm) can be shown, for example, as the ratio of sperms having an average value (path velocity) of the sperm traveling direction velocity of 50 µm/sec or more and a linearity (straightness) of 50% or more to the total number of sperms.

### 2. Experimental example

### (Example 1)

Rat sperms were frozen by (3) Sperm cryopreservation method described above. The temperature change of the sperm suspension from the start of cooling to freezing was measured. The results are shown in FIG. 11. It can be seen that the conventional method of cooling on a petri dish placed on ice can only cool to about 5°C. On the other hand, it was confirmed that the method of the present invention further cooled to about 1°C or lower.

### (Example 2) In vitro fertilization of fresh or cryopreserved rat sperm

In vitro fertilization was performed using fresh sperms and cryopreserved sperms by the method described above. High fertilization rates were achieved with all sperms. The results are shown in Table 1. However, the fertilization rate of cryopreserved sperms was slightly lower than that of fresh sperms.

**[Table 1]**

| Sperm | No. | No. of inseminated oocytes | No. of fertilized oocytes | Fertilization rate (%) |
|---|---|---|---|---|
| Fresh | 1 | 198 | 185 | 93.4 |
| | 2 | 183 | 170 | 92.9 |
| | 3 | 195 | 181 | 92.8 |
| | 4 | 182 | 174 | 95.6 |
| | Total | 758 | 710 | 93.7±1.3 |
| Frozen | 1 | 161 | 135 | 83.9 |
| | 2 | 191 | 160 | 83.8 |
| | 3 | 201 | 161 | 80.1 |
| | 4 | 196 | 159 | 81.1 |
| | Total | 749 | 615 | 82.1±1.9^{∗} |

| | | | | |
|---|---|---|---|---|
| ^{∗}P<0.05 compared with fresh sperm | | | | |

### (Example 3) In vitro developmental ability of rat fertilized oocyte

Fertilized oocytes were cultured in vitro to develop by the method described above. Almost all fertilized oocytes developed into 2-cell stage embryos 28 hours after fertilization. Not less than 60% of 2-cell stage embryos developed in blastocysts, whether derived from cryopreserved sperms or fresh sperms. Half of them were blastocysts with a green fluorescent protein (GFP) signal. The results are shown in FIG. 12.

In addition, the developmental rate from 2-cell stage embryos to blastocysts in vitro is shown in Table 2 below.

**[Table 2]**

| Sperm | No. | No. of 2-cell embryos | No. of blastocysts | Developmental rate (%) | No. of blastocysts with GFP | Success rate (%) |
|---|---|---|---|---|---|---|
| Fresh | 1 | 100 | 58 | 58 | 27 | 46.6 |
| | 2 | 100 | 63 | 63 | 30 | 47.6 |
| | 3 | 100 | 65 | 65 | 33 | 50.8 |
| | 4 | 100 | 71 | 71 | 35 | 49.3 |
| | Total | 400 | 257 | 64.3±5.4 | 125 | 48.6±1.9 |
| Frozen | 1 | 100 | 72 | 72 | 36 | 50.0 |
| | 2 | 100 | 57 | 57 | 28 | 49.1 |
| | 3 | 100 | 64 | 64 | 29 | 45.3 |
| | 4 | 100 | 53 | 53 | 34 | 64.2 |
| | Total | 400 | 246 | 615±5.4 | 127 | 51.6±8.3 |

| | | | | | | |
|---|---|---|---|---|---|---|
| Developmental rate = no. of blastocysts/no. of 2-cell embryos × 100 Success rate = no. of blastocysts with GFP/no. of blastocysts × 100 | | | | | | |

### (Example 3) In vivo developmental ability of rat fertilized oocyte

Fertilized oocytes were developed in vivo by the method described above. A total of 46 normal pups (26 pups with GFP signal: 57%) were obtained from cryopreserved sperms, and a total of 48 normal pups (24 pups with GFP signal: 50%) were obtained from fresh sperms. The results are shown in FIG 13.

In addition, the in vivo developmental rate of fertilized oocytes is shown in Table 3 below.

**[Table 3]**

| Sperm | No. | No. of fertilized oocytes transferred | No. of pups | Birth rate (%) | No. of pups with GFP | Success rate (%) |
|---|---|---|---|---|---|---|
| Fresh | 1 | 20 | 13 | 65.0 | 6 | 46. 2 |
| | 2 | 20 | 12 | 60.0 | 5 | 41.7 |
| | 3 | 20 | 13 | 65.0 | 6 | 46.2 |
| | 4 | 20 | 10 | 50.0 | 7 | 70.0 |
| Frozen | Total | 80 | 48 | 60±7.1 | 24 | 50.0±12.8 |
| | 1 | 20 | 12 | 60.0 | 8 | 66.7 |
| | 2 | 20 | 12 | 60.0 | 7 | 58.3 |
| | 3 | 20 | 10 | 50.0 | 2 | 20.0 |
| | 4 | 20 | 12 | 60.0 | 9 | 75.0 |
| | Total | 80 | 46 | 57.5±5.0 | 26 | 56.5±24.3 |

| | | | | | | |
|---|---|---|---|---|---|---|
| **Birth rate = no. of pups/no. of fertilized oocytes transferred** **×100 Success rate = no. of pups with GFP/no. of pups × 100** | | | | | | |

### (Example 4) Refrigeration preservation of cauda epididymis

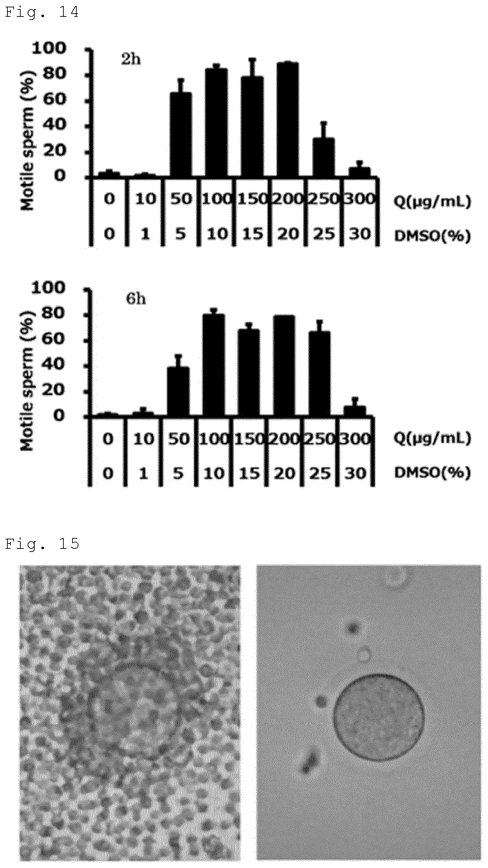

After removing the cauda epididymis from a rat, it was preserved under refrigeration at 4°C for 3 days using a refrigeration preservation solution supplemented with various concentrations of dimethyl sulfoxide and quercetin. Then, a sperm suspension was prepared from the cauda epididymis, and the proportion of sperms in movement in the prepared sperm suspension was measured. The results are shown in FIG. 14. It was found that rat sperms can be preserved under refrigeration by adding dimethyl sulfoxide and quercetin in combination. In particular, good results were obtained by combining 5 to 25% dimethyl sulfoxide with 50 to 250 µg/mL quercetin.

The rat cauda epididymis was preserved under refrigeration for 1 to 3 days using a Lifor^{®} refrigeration preservation solution supplemented with 5% dimethyl sulfoxide and 100 µg/mL quercetin, then, a sperm suspension was prepared and in vitro fertilization was performed. As the in vitro fertilization medium used, an mHTF medium and a medium containing 40 mg/mL of BSA added to this (referred to as Rat IVF medium) were used. The results of fertilization rate are shown in the table below.

**[Table 4]**

| | **Fertilization rate** | | |
|---|---|---|---|
| **Medium** | **Day 1 of refrigiration** | **Day 2 of refrigiration** | **Day 3 of refrigiration** |
| **mHTF medium** | **42/52 (81%)** | **44/67 (66%)** | **74/87 (85%)** |
| **Rat IVF medium** | **35/79 (44%)** | **77/101 (76%)** | **110/113 (97%)** |

| | | | |
|---|---|---|---|
| **Number of oocyte used in in vitro fertilization/number of fertilized oocyte (fertilization rate (%))** | | | |

The fertilization medium (Rat IVF medium) supplemented with BSA provided a good fertilization rate even with a longer refrigeration period.

### (Example 5) In vitro fertilization using frozen sperm prepared from refrigerated cauda epididymis

The rat cauda epididymis was preserved under refrigeration for 2 or 3 days, and then frozen sperms were prepared, according to the method described in (4) above. As the refrigeration preservation solution, a Lifor^{®} refrigeration preservation solution supplemented with 5% dimethyl sulfoxide and 100 µg/mL quercetin was used. Frozen sperms were thawed according to the method described in (5) above. The proportion of sperms having motility after thawing was from about 1% to about 2%. Then, in vitro fertilization was performed according to the method described in (6) above. As the in vitro fertilization medium, an mHTF medium containing 40 mg/mL BSA was used, and as the oocyte, an oocyte obtained by treating with 0.1% hyaluronidase to remove the cumulus oophorus was used. Photographs of a normal oocyte (untreated oocyte) and an oocyte from which the cumulus oophorus has been removed (cumulus oophorus-removed oocyte) are shown in FIG. 15. Fertilization rate (%) (number of oocytes used for in vitro fertilization/number of fertilized oocytes) was 47% (21/45) for 2-day refrigerated frozen sperms and 32% (11/34) for 3-day refrigerated frozen sperms, thus, it was found that a good fertilization rate can be achieved by using the method of the present invention even when frozen sperms prepared from sperms preserved under refrigeration are used.

### (Example 6) In vitro fertilization using frozen sperm of various strain rat

When in vitro fertilization was performed in the same manner as in Example 2 using F344 strain rats instead of SD strain rats, the fertilization rate (%) (number of oocytes used for in vitro fertilization/number of fertilized oocytes) showed a very low fertilization rate of 2.9% (2/70) . Then, in vitro fertilization was carried out in the same manner using an mHTF medium supplemented with 40 mg/mL BSA (Rat IVF medium), instead of mHTF, as the in vitro fertilization medium, and further using oocytes from which cumulus oophorus had been removed. The results for each condition are shown in the table below.

**[Table 5]**

| | **Fertilization rate** | |
|---|---|---|
| **Medium** | **mHTF medium** | **Rat IVF medium** |
| **Untreated oocyte** | **2/70 (2.9%)** | **23/61 (37.7%)** |
| **Cumulus oophorus-removed oocyte** | | **33/44 (75.0%)** |

| | | |
|---|---|---|
| **Number of oocyte used in in vitro fertilization/number of fertilized oocyte (fertilization rate (%))** | | |

By using the oocytes from which the cumulus oophorus had been removed and the medium supplemented with BSA, a good fertilization rate could be achieved even when the F344 strain rats were used.

The results of the case of performing in vitro fertilization using rats of various strains under the normal condition described in Example 2 (untreated oocyte, mHTF medium) (Condition A) and the condition described in Example 6 (cumulus oophorus-removed oocyte, Rat IVF medium (mHTF medium containing 40 mg/mL BSA)) (Condition B) are shown below. In the table, double circles have a fertilization rate of 50% or more, Δ has a fertilization rate of 10 to 50%, and × has a fertilization rate of less than 10%.

**[Table 6]**

| | **Fertilization rate** | |
|---|---|---|
| **Rat Strain** | **Condition A** | **Condition B** |
| **CD(SD)** | **⊚** | **⊚** |
| **Wister** | **⊚** | **⊚** |
| **Long-Evans** | **Δ** | **⊚** |
| **BN** | **×** | **A** |
| **F344** | **×** | **⊚** |

Sufficient fertilization rate could be achieved in any of the strains by using the oocyte from which the cumulus oophorus had been removed and the medium supplemented with BSA.

### (Example 6) In vitro fertilization using frozen sperm of SD strain and Wistar strain rat

The fertilization rate was measured in in vitro fertilization using cumulus oophorus-removed oocytes in an mHTF medium containing BSA, which is a preferable condition, using frequently used SD and Wistar strain rats according to the method of the present invention. The results are shown in the table below.

**[Table 7]**

| | **Rat Strain** | **SD(CD)** | **Wistar** |
|---|---|---|---|
| **Type of oocyte** | **In vitro fertilization medium (mHTF medium)** | **Fertilization rate** | **Fertilization rate** |
| **Untreated oocyte** | **BSA: 4mg/ml** | **142/195 (72.8%)** | **160/214 (74.8%)** |
| **Untreated oocyte** | **BSA: 40mg/ml** | **121/124 (97.6%)** | **130/137 (94.9%)** |
| **Cumulus oophorus-removed oocyte** | **BSA: 40mg/ml** | **182/190 (95.8%)** | **173/185 (93.5%)** |

| | | | |
|---|---|---|---|
| **Number of oocyte used in in vitro fertilization/number of fertilized oocyte (fertilization rate (%))** | | | |

It was shown that a fertilization rate of more than 90% can be achieved in vitro fertilization using untreated oocytes and cumulus oophorus-removed oocytes in an mHTF medium containing BSA according to the method of the present invention.

According to the above results, fertilized oocytes derived from either cryopreserved sperms or fresh sperms were similarly developed both in vivo and in vitro. From this, it was shown that the rat sperm cryopreservation method of the present invention is effective.

The foregoing merely illustrates objects and subjects of the present invention, and is not intended to be limiting the accompanying Claims. Without departing from the accomp anying Claims, various modifications and alterations to the described embodiments will be apparent to those skilled in t he art in view of the teachings herein.

### [Industrial Applicability]

The method of the present invention provides a practically useful method for cryopreserving and thawing rat sperms and an in vitro fertilization method using cryopreserved rat sperms.

## Claims

1. A method for preparing cryopreserved rat sperms, **characterized by** comprising the following steps:
Step a: A preparation step of collecting rat sperms from the rat cauda epididymis to prepare a sperm suspension,
Step b: A cooling step of cooling the sperm suspension to about 1°C or lower, and
Step c: A freezing step of freezing the rat sperm suspension cooled to about 1°C or lower.

2. The method according to Claim 1, wherein the cooling step of said step b is performed in order to substantially reduce the movement of rat sperms.

3. The method according to Claim 1 or 2, wherein said step b comprises a two-stage process of (b-1) a step of cooling the rat sperm suspension to about 4°C to about 6°C, and (b-2) a step of further cooling the primarily cooled sperm suspension to about 1°C or lower.

4. The method according to Claim 3, wherein at least said step (b-2) is performed in a cryopreservation container for cryopreserving sperms.

5. The method according to Claim 3 or 4, wherein said step (b-2) is performed by placing the cryopreservation container containing the sperm suspension on ice for about 15 minutes to about 45 minutes.

6. The method according to any one of Claims 1 to 5, wherein in said step b, the motion capability of the sperms after cooling is 20% or less as compared with the motion capability of the sperms before cooling.

7. The method according to any one of Claims 1 to 5, wherein in said step b, the motion rate of the sperms after cooling is 50% or less as compared with the motion rate of the sperms before cooling.

8. The method according to any one of Claims 1 to 7, wherein said rat cauda epididymis is rat cauda epididymis stored under refrigeration.

9. The method according to any one of Claims 1 to 8, wherein said rat sperm is a sperm derived from a gene-modified rat.

10. Use of the cryopreserved rat sperms prepared by the method as described in any one of Claims 1 to 9, for in vitro fertilization.

11. A method for preparing rat sperms for use in in vitro fertilization, **characterized by** comprising the following steps:
Step A: A thawing step of heating a cryopreservation solution containing frozen rat sperms to a temperature of about 35°C to about 37.5°C to prepare a thawed rat sperm suspension,
Step B: A swim-up step of placing said thawed rat sperm suspension at a lower portion of the medium contained in a container and allowing it to stand to swim up the sperms,
Step C: A first recovery step of recovering sperms, and
Step D: A second recovery step of transferring the recovered sperms to a sperm culture solution and recovering sperms with high motility.

12. The method according to Claim 11, wherein the lower portion of the container in said step B is a tube having a tapered or conical tip.

13. The method according to Claim 11 or 12, wherein the medium in said step B has a volume of about 5 to about 20 times that of the sperm suspension.

14. The method according to any one of Claims 11 to 13, wherein the standing in said step B is conducted for about 20 to about 40 minutes.

15. The method according to any one of Claims 11 to 14, wherein the step C is a step of recovering sperms by mixing the thawed rat sperm suspension and the medium in a container and then centrifuging at a low speed to recover the precipitate.

16. Use of the rat sperms prepared by the method as described in any one of Claims 11 to 15, for in vitro fertilization.

17. An in vitro fertilization method, comprising the following steps:
(i) a step of thawing the cryopreserved rat sperms prepared by the method as described in any one of Claims 1 to 9, using the method as described in any one of Claims 11 to 15, and preparing them for in vitro fertilization, and then pre-culturing the prepared rat sperms in a sperm preculture medium (wherein, the sperm is pre-cultured in a medium used for in vitro fertilization prior to in vitro fertilization),
(ii) a step of administering horse chorionic gonadotropin (eCG) to female rats and then human chorionic gonadotropin (hCG), and then collecting unfertilized oocytes to prepare unfertilized oocytes, and
(iii) a step of mixing the sperms pre-cultured in the step (i) with the unfertilized oocytes prepared in the step (ii) to perform insemination.

18. The in vitro fertilization method according to Claim 17, wherein said step (ii) further includes a cumulus removal step of removing the cumulus oophorus of the prepared unfertilized oocyte.

19. The in vitro fertilization method according to Claim 17 or 18, wherein the sperm preculture medium in said step (i) is an mHTF medium containing about 1 to about 80 mg/mL of bovine serum albumin.

20. A rat sperm preculture medium for use in pre-culturing rat sperms for in vitro fertilization using cryopreserved rat sperms, comprising an mHTF medium or TYH medium containing about 20 mg/mL to about 80 mg/mL of bovine serum albumin.

21. A rat in vitro fertilization medium for use in in vitro fertilization using cryopreserved rat sperms, comprising an mHTF medium or TYH medium containing about 30 to about 50 mg/mL of bovine serum albumin.
